# EUROPÄISCHE PATENTANMELDUNG

(11) **EP 0 651 976 A1**
(43) Veröffentlichungstag der Anmeldung: **10.05.1995**
(21) Anmeldenummer: 94113481.9
(22) Anmeldetag: 29.08.1994
(51) Int. Cl.: A61C 8/00

(54) **Vorrichtung zur Befestigung eines Zahnersatzes im menschlichen Kiefer**

(30) Priorität: 21.10.1993 DE 9316043 U
(71) Anmelder: Brammann, Dierk, 22305 Hamburg (DE)
(72) Erfinder: Brammann, Dierk, 22305 Hamburg (DE)
(74) Vertreter: Dipl.-Ing. H. Hauck, Dipl.-Ing. E. Graalfs, Dipl.-Ing. W. Wehnert, Dr.-Ing. W. Döring, Dr.-Ing. N. Siemons

(57) **Zusammenfassung**

Vorrichtung zur Befestigung eines Zahnersatzes im menschlichen Kiefer, mit einem im Kiefer zu inserierenden Implantat (10), das als geschlossene Hülse geformt ist, einem einen Verbindungsschaft (28) aufweisenden, mit dem Implantat verbindbaren Sekundärteil (24), das Träger für den Zahnersatz (43) ist und einem Befestigungselement (40) zur Festlegung des Zahnersatzes am Sekundärteil, wobei der Verbindungsschaft (28) in der Sackbohrung (14) des Implantats (10) sitzt und am proximalen Ende mindestens einen achsparallelen Schlitz (30) aufweist zur Bildung einer Schnapp- und Spreizverbindung mit einem Hinterschnitt (34) in der Sackbohrung, wobei der Schaft einen radial spreizbaren Abschnitt (32) aufweist, der mit Hilfe eines über eine axiale Bohrung im Sekundärteil eingesetzten Dorns (40) aufgespreizt bzw. radial im Implantat festgelegt werden kann und das Sekundärteil eine radiale Schulter aufweist, die sich auf dem distalen Ende des Implantats abstützt, wenn der aufspreizbare Abschnitt hinter den Hinterschnitt greift.

## Beschreibung

Die Erfindung bezieht sich auf eine Vorrichtung zur Befestigung eines Zahnersatzes im menschlichen Kiefer nach dem Oberbegriff des Anspruchs 1.

Es ist bekannt, Zahnprothesen, wie Kronen, Brücken oder dergleichen mit Hilfe sogenannter Implantate im Kiefer festzulegen. In den Kiefer wird ein Implantat aus körperverträglichem Material, zum Beispiel Titan, inseriert und über einen längeren Zeitraum festwachsen gelassen. Das Implantat ist normalerweise hülsenartig oder weist zumindest distal eine Innenbohrung auf zur Aufnahme eines Sekundärteils, das dann als Träger für die Zahnprothese dient. Es ist bekannt, das Sekundärteil mit dem Implantat (Primärteil) zu verschrauben und die Prothese ihrerseits mit dem Sekundärteil zu verschrauben.

Aus der DE 33 00 764 ist ein Implantat bekanntgeworden, bei dem der Implantatkörper eine distale Ringschulter aufweist und ein mit der Ringschulter koaxiales Sackloch, in das ein Schaft des Sekundärteils eingreift. Der Aufsatz des Sekundärteils stützt sich über einen Kragen und einen Distanzring auf der Ringschulter ab. Der Distanzring ist als elastisch verformbare Ringlippe ausgebildet. Das Sackloch endet in einer Aushöhlung, die tief im Inneren des Implantatkörpers liegt. Der Zapfen trägt einen in die Aushöhlung greifenden Spannkopf, der elastisch gegeneinander verformbare Segmente aufweist, wodurch der Kopf bis auf mindestens den Mündungsquerschnitt des Sackloches elastisch zusammengedrückt werden kann und sich in der Aushöhlung so weit aufspreizt, daß sich die Spannkopfsegmente an der Wand der Aushöhlung im oberen und unteren Teil des Kopfes anlegen. Auf diese Weise ist das Sekundärteil begrenzt axial und zur Seite beweglich im Implantat abgestützt.

Der Erfindung liegt die Aufgabe zugrunde, eine Vorrichtung zur Befestigungs eines Zahnersatzes im menschlichen Kiefer zu schaffen, die eine präzise Ausformung und Anbringung des Zahnersatzes ermöglicht. Außerdem soll eine stabile Position des Zahnersatzes gewährleistet sein.

Diese Aufgabe wird durch die Merkmale des Patentanspruchs 1 gelöst.

Bei der erfindungsgemäßen Vorrichtung stützt sich das Sekundärteil über eine radiale Schulter auf dem proximalen Ende des Implantats ab, sorgt mithin für eine stabile Lagerung des Zahnersatzes in axialer Richtung. Die entgegengesetzt wirkenden Kräfte in Querrichtung und in Auszugsrichtung werden durch den Schaft innerhalb der Implantathülse aufgefangen. Mit Hilfe des Dorns wird der Schaft im Endbereich radial aufgeweitet, so daß er mit dem Hinterschnitt zusammenwirkt und fest in der Implantathülse gehalten ist. Einer Auszugskraft ist dadurch ein unüberwindbarer Widerstand entgegengesetzt.

Es ist denkbar, den Hinterschnitt durch eine konische Fläche in der Bohrung zu bilden, die von einer konischen Fläche an der Außenseite der aufspreizbaren Segmente des Schaftes gebildet werden. Es ist jedoch nach einer Ausgestaltung der Erfindung zu bevorzugen, wenn der aufspreizbare Abschnitt des Schaftes eine zur Längsachse geneigte ringförmig umlaufende Außenfläche aufweist, die mit einer ringförmig umlaufenden Gegenfläche in der Bohrung des Implantats so zusammenwirkt, daß bei einer radialen Spreizung des geschlitzten Abschnitts das Sekundärteil axial in das Innere der Bohrung hineingezogen wird. Beim Einführen des Dorns, beispielsweise durch Einschrauben in die Bohrung des Schaftes, wird der Schaft bzw. das Sekundärteil axial in Richtung der Implantathülse vorgespannt, so daß das Sekundärteil mit seiner Schulter fest mit der Implantathülse zur Anlage gebracht werden kann. Auf diese Weise läßt sich ein definierter Randspalt zwischen Sekundärteil und Implantat herstellen.

Es ist besonders vorteilhaft, wenn der geschlitzte bzw. aufspreizbare Abschnitt des Schaftes kugelförmig ist und mit dem kugelförmigen Abschnitt einen konvexen Wulst in der Bohrung des Implantats untergreift. Bei fortschreitendem Aufspreizen mit Hilfe des Dorns wird der Schaft weiter in das Innere der Bohrung des Implantats hineingezogen und preßt mithin die radiale Schulter des Sekundärteils gegen das äußere Ende des Implantats. Der Schaft kann so ausgebildet sein, daß er über eine größere Länge annähernd passend in der Sackbohrung des Implantats einsitzt, um Querkräfte aufzufangen. Durch die beschriebene kugelförmige Ausbildung des freien Endes des Schaftes wird der Anlagebereich des Schaftes sehr nahe an das Ende der Sackbohrung gelegt, so daß ein großer Hebelarm zur Aufnahme von Querkräften gebildet ist.

Durch eine entsprechende Ausformung der Querschnitte von Sekundärteil bzw. Schaft einerseits und einem Bohrungsabschnitt des Implantats andererseits läßt sich das Sekundärteil drehgesichert im Implantat anbringen.

Es ist bekannt und üblich, den sogenannten Aufsatz des Sekundärteils, d.h. den aus dem Implantat herausstehende Abschnitt des Sekundärteils, konisch zu formen. Nach einer Ausgestaltung der Erfindung wird eine konische Kappe vorgesehen, die passend auf das freiliegende Ende des Sekundärteils aufgesteckt wird. Vorzugsweise weist das freiliegende Ende einen Konus auf, der die konische Kappe passend aufnimmt. Die Verwendung einer konischen Kappe hat den Vorteil, daß verschiedene Kappen mit verschiedenen Außenabmessungen und Winkeln verwendet werden können, die je nach Anwendungsfall auf den konischen Abschnitt des Sekundärteils aufgesteckt werden.

Die Erfindung wird nachfolgend anhand von Zeichnungen näher erläutert.
- Fig. 1: zeigt einen Schnitt durch eine erste Ausführungsform der Vorrichtung nach der Erfindung.
- Fig. 2: zeigt einen Schnitt durch eine zweite Ausführungsform der Vorrichtung nach der Erfindung.

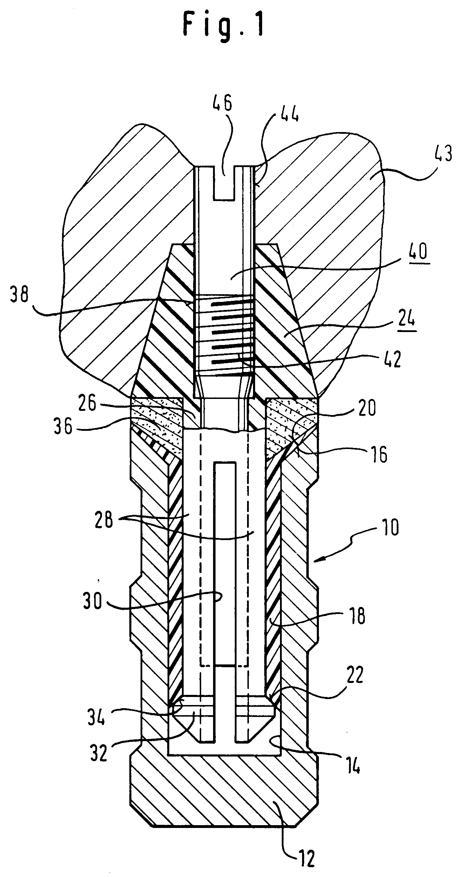

In Fig. 1 ist ein Implantat 10 aus einem geeigneten körperverträglichen Material, zum Beispiel Titan, in herkömmlicher Weise hülsenartig ausgebildet mit proximal geschlossenem Ende 12. Das Implantat 10 weist eine zylindrische Sackbohrung 14 auf und ist marginal bzw. oral mit einer konischen Stirnfläche 16 versehen. In die Sackbohrung 14 ist eine zylindrische Hülse 18 eingesetzt und darin befestigt, die zum Beispiel aus dem gleichen Material wie das Implantat 10 bestehen kann oder aus einem geeigneten körperverträglichen Kunststoff, wie Polymethylacrylat oder einem HDPE (hochdichtem Polyethylen). Auch die Hülse 18 ist mit einer konischen Stirnfläche 20 versehen, die mit der Stirnfläche 16 fluchtet. Die Hülse 18 ist kürzer als die Sackbohrung 14, so daß sich ein Hinterschnitt 22 ergibt, der ebenfalls konisch ausgebildet ist.

Ein konisches Sekundärteil 24 weist einen im Durchmesser geringeren Schaft 26 auf, der zum Beispiel aus vier parallelen Beinen 28 besteht, die durch achsparallele Längsschlitze 30 voneinander getrennt sind. Jedes Bein 28 weist am unteren Ende eine Verdickung 32 auf mit einer oberen angeschrägten Fläche 34, die mit der entsprechend ausgebildeten Gegenfläche 22 der Hülse 18 zusammenwirkt.

Wie erkennbar, erstreckt sich der Schaft 26 in die Hülse 18 hinein, wobei die verdickten Abschnitte 32 sich in dem Raum unterhalb der Enden der Hülse 18 erstrecken. Der Schaft 28 durchsetzt außerdem einen nach proximal konisch ausgebildeten Ring 36 aus einem geeigneten körperverträglichen Material, das sich nach oben gegen die kreisringartige Unterseite des Sekundärteils 24 anlegt. Der Konus des Rings 36 wirkt passend mit der konischen Stirnfläche 16 des Implantats 10 bzw. der konischen Stirnfläche 20 der Hülse 18 zusammen. Es kann auch daran gedacht sein, den Ring 36 aus einem nachgebenden Material zu formen.

Durch die hindurchgehende Bohrung 38 des Sekundärteils 24 erstreckt sich ein länglicher Dorn 40. Der Dorn hat einen Gewindeabschnitt und wirkt mit einem Gewindeabschnitt 42 der Bohrung 38 zusammen. Auf dem Sekundärteil 24 sitzt passend eine Krone 42 mit einer durchgehenden Bohrung 44, durch die sich der Dorn 40 hindurcherstreckt, wobei er jedoch deutlich aus dem Sekundärteil 24 herausragt. Der Dorn 40 weist am offenen Ende einen Schraubenzieherschlitz 46 auf, damit er durch ein geeignetes Werkzeug verdreht werden kann.

Bei der Anbringung am Kiefer wird zunächst das Sekundärteil 24 zusammen mit dem aufgeschobenen Ring 36 in das Implantat 10 eingesteckt, bis die Verdickungen 32 hinter die Stirnfläche 22 der Hülse 18 rasten. Anschließend wird die Krone 42 auf das Sekundärteil 24 aufgesetzt und danach der Dorn 40 durch die Krone, das Sekundärteil 24, den Ring 36 und die Hülse 18 hindurchgeführt und verschraubt. Durch die Verschraubung kann eine Zugspannung zwischen dem Sekundärteil 24 und dem Implantat 10 ausgeübt werden zur sicheren Befestigung des Sekundärteils 24 und Implantat 10 unter Pressung des Rings 36. Dadurch ist der Zahnersatz 42 fest am Implantat 10 angebracht.

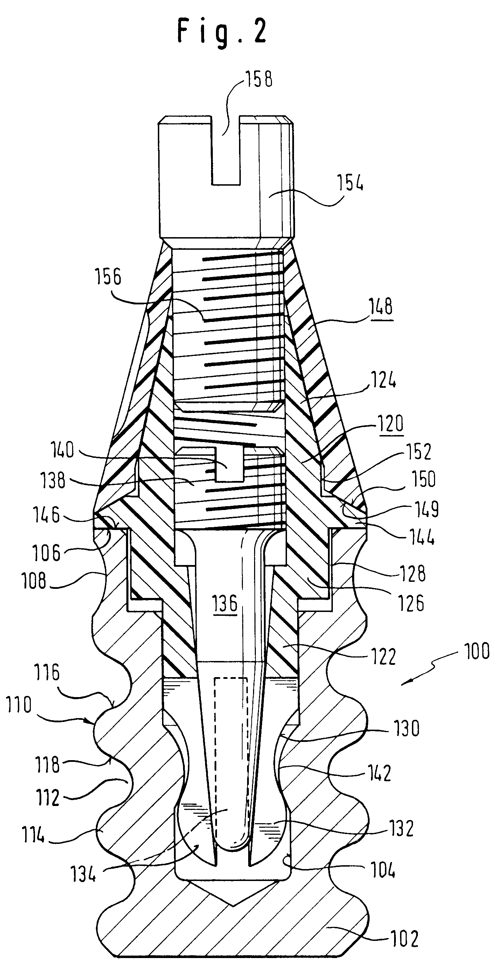

Bei der Ausführungsform nach Fig. 2 ist wiederum ein hülsenförmiges Implantat 100 zu erkennen, das am proximalen Ende 102 geschlossen ist. Sie weist eine Sackbohrung 104 auf nahe dem oberen Ende 106, das als plane Ringfläche senkrecht zur Achse des Implantats 100 geformt ist. An der Außenseite des Implantats 100 ist eine im Querschnitt kreisförmige Ringnut 108 geformt. Unterhalb der Ringnut 108 ist ein Gewinde 110 geformt, das eine im Querschnitt gerundete Gewindenut 112 und einen gerundeten Gewindegang 114 aufweist. Die nach oben weisenden Flanken 116 sind steiler als die entgegengesetzten Flanken 118, so daß das Eindrehen der Implantathülse 100 in eine vorgeformte Bohrung im Kiefer relativ leicht vonstatten geht, jedoch hohe Auszugskräfte erhalten werden. Die Bohrung hat einen Durchmesser, der gleich ist dem Kerndurchmesser des Gewindes 110. Das Implantat 100 kann mithin zementlos im Kiefer inseriert werden.

Ein Sekundärteil 120 besitzt einen in der Sackbohrung 104 eingeführten Schaft 122 sowie einen Aufbau 124 außerhalb des Implantats 100. Wie aus Fig. 2 zu erkennen, sitzt der Schaft 122 über eine größere Länge passend in der Bohrung 104, wobei ein im Durchmesser erweiterter Abschnitt 126 in einem erweiterten Bohrungsabschnitt 128 sitzt. Der Abschnitt 126 ist im Querschnitt sechseckförmig. Dementsprechend hat auch die Ausnehmung 128 Sechseckform. Dadurch ist der Schaft 122 gegen Drehung gesichert.

Im unteren oder proximalen Bereich weist der Schaft 122 eine umlaufende im Querschnitt kreisförmige Nut 130 auf, die eine Einschnürung bildet. Unterhalb der Einschnürung 130 ist ein kugelförmiger Abschnitt 132 vorgesehen, der durch achsparallele Schlitze 134 in vier um 90° versetzte Segmente unterteilt ist.

Das Sekundärteil 120 weist eine durchgehende axiale Bohrung auf, die im oberen Bereich ein Innengewinde aufweist. Ein Dorn 136, der im unteren Bereich konisch geformt ist, weist einen Kopf 138 mit einem Außengewinde auf, das in das Innengewinde der durchgehenden Bohrung eingeschraubt werden kann. Der Kopf 138 weist einen Schraubenzieherschlitz 140 auf, damit der Dorn 136 in das Sekundärteil 120 eingeschraubt werden kann.

Die Sackbohrung 104 hat einen radial nach innen weisenden konvexen umlaufenden Wulst 142, der einen Hinterschnitt bildet für den kugelförmigen Abschnitt 132 des Schaftes 122. Das Sekundärteil 120 weist einen radial nach außen weisenden ringförmigen Flansch 144 auf, der eine nach unten weisende ringförmige Fläche oder Schulter 146 bildet. Gegenüberliegend weist der Flansch eine konische Fläche 148 auf. Wird der Schaft 122 in die Sackbohrung 104 eingeführt, können die Segmente des kugelförmigen Abschnitts 132 nach innen ausweichen und dabei den Wulst 142 passieren. Anschließend spreizen sie sich und legen sich an der Unterseite des Wulstes 142 an, wenn die Schulter 146 mit der Stirnfläche 106 des Implantats 100 zur Anlage kommt. Wird nun der Dorn 138 eingeschraubt, spreizt er die Segmente des kugelförmigen Abschnitts 132 radial nach außen, wodurch der Schaft 122 in der Bohrung 104 axial festgelegt wird. Die Ausbildung der Segmente des kugelförmigen Abschnitts 132 und des Wulstes 142 ist derart, daß beim Aufspreizen der Segmente gleichzeitig eine axiale Kraft in Richtung des Endes der Sackbohrung 104 erzeugt wird, wodurch die Flächen 106, 146 fest gegeneinandergepreßt werden und dadurch einen definierten Spalt bilden. Es sei nebenbei bemerkt, daß der Dorn 136 keineswegs konisch geformt zu sein braucht.

Auf dem konischen Aufbau 124 ist eine Kappe 148 aufgeschoben, die innen und außen konisch geformt ist. Der Innenkonus der Kappe 148 paßt zum Außenkonus des Aufbaus 124. Das in Fig. 2 untere Ende der Kappe 148 ist konisch, wie bei 150 gezeigt, wobei die konische Fläche mit der konischen Fläche 148 des Ringflansches 144 passend zusammenwirkt. Aufbau 124 und Kappe 148 sind im Querschnitt kreisförmig, wobei jedoch an mindestens einer, vorzugsweise an vier um 90° beabstandeten Positionen Abflachungen an der Außenseite des Aufbaus 124 und der Innenseite der Kappe 148 geformt sind, wie bei 152 angedeutet, um die Kappe 148 drehfest am Aufbau 124 festzulegen.

Wie erkennbar, erstreckt sich die Kappe 148 in Fig. 2 nach oben über den Aufbau 124 hinaus und wird durch den Kopf 154 einer Schraube 156, die in die Gewindebohrung des Sekundärteils 120 eingeschraubt ist, am Sekundärteil festgelegt. Die Schraube 156 weist wiederum einen Schlitz 158 auf für einen Schraubendreher. Die Länge des Schaftes der Schraube 156 ist derart, daß in jedem Fall ein Abstand zum Kopf 138 des Dorns 136 besteht, wenn der Dorn in seiner endgültigen Position ist.

Das Implantat 100 besteht aus einem geeigneten körperverträglichen Metall. Die übrigen Teile bestehen aus einem geeigneten Metall oder Kunststoffmaterial, wie es etwa auch für die Vorrichtung nach Fig. 1 verwendet wird.

## Patentansprüche

**1.** Vorrichtung zur Befestigung eines Zahnersatzes im menschlichen Kiefer, mit einem im Kiefer zu inserierenden Implantat, das als geschlossene Hülse geformt ist, einem einen Verbindungsschaft aufweisenden, mit dem Implantat verbindbaren Sekundärteil, das Träger für den Zahnersatz ist und einem Befestigungselement zur Festlegung des Zahnersatzes am Sekundärteil, wobei der Verbindungsschaft in der Sackbohrung des Implantats sitzt und am proximalen Ende mindestens einen achsparallelen Schlitz aufweist zur Bildung einer Schnapp- und Spreizverbindung mit einem Hinterschnitt in der Sackbohrung, dadurch gekennzeichnet, daß der Schaft (26, 120) einen radial spreizbaren Abschnitt (32, 132) aufweist, der mit Hilfe eines über eine axiale Bohrung im Sekundärteil (24, 124) eingesetzten Dorns (40, 136) aufgespreizt bzw. radial im Implantat (10, 110) festgelegt werden kann und das Sekundärteil (24, 120) eine radiale Schulter (146) aufweist, die sich auf dem distalen Ende (106) des Implantats (10, 110) abstützt, wenn der aufspreizbare Abschnitt (32, 132) hinter den Hinterschnitt (34, 142) greift.

**2.** Vorrichtung nach Anspruch 1, dadurch gekennzeichnet, daß der Hinterschnitt (34) durch eine separat gefertigte, in die Bohrung (14, 104) eingesetzte Innenhülse (18) gebildet ist.

**3.** Vorrichtung nach Anspruch 1 oder 2, dadurch gekennzeichnet, daß der Dorn (26, 136) ein Außengewinde aufweist, das mit einem Innengewinde im Sekundärteil (24, 120) zusammenwirkt.

**4.** Vorrichtung nach einem der Ansprüche 1 bis 3, dadurch gekennzeichnet, daß der Dorn (40) zugleich zur Befestigung des Zahnersatzes (42) am Sekundärteil (24) dient.

**5.** Vorrichtung nach einem der Ansprüche 1 bis 4, dadurch gekennzeichnet, daß der Dorn (136) innerhalb des Sekundärteils (120) versenkt ist und Platz läßt für eine in das Sekundärteil (120) einsetzbare Schraube (156) zur Festlegung des Zahnersatzes.

**5.** Vorrichtung nach einem der Ansprüche 1 bis 5, dadurch gekennzeichnet, daß der geschlitzte bzw. aufspreizbare Abschnitt (132) des Schaftes (122) eine zur Längsachse geneigte ringförmig umlaufende Außenfläche aufweist, die mit einer ringförmig umlaufenden Gegenfläche (142) in der Bohrung (104) des Implantats (100) so zusammenwirkt, daß bei einer radialen Spreizung des geschlitzten Abschnitts (132) das Sekundärteil (120) axial in das Innere der Bohrung (104) hineingezogen wird.

**7.** Vorrichtung nach Anspruch 6, dadurch gekennzeichnet, daß der geschlitzte Abschnitt (132) im Abstand vom Ende eine Einschnürung (130) aufweist und das Ende von einem kugelförmigen Abschnitt (132) gebildet ist und die Bohrung (104) einen radial nach innen weisenden konvexen Wulst (142) aufweist, hinter den der kugelförmige Abschnitt (132) greift.

**8.** Vorrichtung nach einem der Ansprüche 1 bis 7, dadurch gekennzeichnet, daß der Schaft (122) drehgesichert in der Bohrung (104) des Implantats (100) sitzt.

**9.** Vorrichtung nach Anspruch 8, dadurch gekennzeichnet, daß die Bohrung (104) des Implantats (100) am offenen Ende eine im Durchmesser erweiterte Ausnehmung (128) aufweist, in der ein entsprechend geformter Abschnitt (126) des Sekundärteils (120) sitzt und der Umfang von Ausnehmung (128) und Abschnitt (126) von der Kreisform abweicht.

**10.** Vorrichtung nach Anspruch 9, dadurch gekennzeichnet, daß die Ausnehmung (128) und der in der Ausnehmung (128) sitzende Abschnitt (126) sechseckig sind.

**11.** Vorrichtung nach einem der Ansprüche 1 bis 10, dadurch gekennzeichnet, daß der außerhalb des Implantats (100) liegende Abschnitt (124) des Sekundärteils (120) eine konische Außenfläche hat und eine Kappe (148) vorgesehen ist mit konischer Innen- und Außenfläche, wobei die Innenfläche passend auf dem konischen Abschnitt (124) sitzt.

**12.** Vorrichtung nach Anspruch 11, dadurch gekennzeichnet, daß die Kappe (148) drehgesichert auf dem konischen Abschnitt (124) des Sekundärteils (120) sitzt.

**13.** Vorrichtung nach Anspruch 5 und 11, dadurch gekennzeichnet, daß die Kappe (148) mit Hilfe eines Kopfes (154) einer Schraube (156) am Sekundärteil (120) festgelegt ist.

**14.** Vorrichtung nach einem der Ansprüche 1 bis 13, dadurch gekennzeichnet, daß die Kappe (148) gegen einen radialen Flansch (144) des Sekundärteils (120) anliegt und diesen abdeckt, wobei der Flansch (144) die radiale Schulter (146) bildet.
